# EUROPÄISCHE PATENTSCHRIFT

(11) **EP 0 891 763 B1**
(45) Veröffentlichungstag und Bekanntmachung des Hinweises auf die Patenterteilung: **22.01.2003**
(21) Anmeldenummer: 98113232.7
(22) Anmeldetag: 16.07.1998
(51) Int. Cl.: A61K 6/10

(54) **Abformmaterial auf Silikonbasis**
Silicone dental impression material
Composition pour la prise d'empreintes dentaires à base de silicones

(30) Priorität: 16.07.1997 DE 19730515
(43) Veröffentlichungstag der Anmeldung: 20.01.1999
(73) Patentinhaber: 3M ESPE AG, 82229 Seefeld (DE)
(72) Erfinder: Wanek, Erich Dr., 86916 Kaufering (DE); Zech, Joachim Dr., 82229 Hechendorf (DE)
(74) Vertreter: Freiherr von Wittgenstein, Arved, Dr.

(56) Entgegenhaltungen:
- EP-A- 0 480 238
- WO-A-96/26246
- DE-A- 4 129 613
- GB-A- 2 159 167

## Beschreibung

Die vorliegende Erfindung betrifft dentale Abformmassen auf der Basis additionsvernetzender Silikone. Die erfindungsgemäßen Abformmassen zeichnen sich durch eine nach ISO 4823 bestimmte mittlere Konsistenz im angemischten Zustand, gute Hydrophilie und gutes Fließvermögen bei gleichzeitig gegebener hervorragender Standfestigkeit aus. Die erfindungsgemäßen Rezepturen sind insbesondere für die maschinelle Anmischung in automatischen Mischgeräten (z.B. Pentamix®, Fa. ESPE, Seefeld) geeignet. Die Materialien werden als sogenannte Monophasen-Abformmaterialien eingesetzt und können in der Praxis aufgrund ihres Fließverhaltens und ihrer guten Beschneidbarkeit als Ersatz für Alginate eingesetzt werden.

In der zahnärztlichen Praxis werden häufig Abformmaterialien auf der Basis von Alginaten zur Situationsabformung von Zähnen und Kieferanteilen zur Auswertung, Diagnose, Planung und Kontrolle der Paßgenauigkeit konservierender, prothetischer und kieferorthopädischer Arbeiten eingesetzt. Hierbei wird die Grobform des Kiefers und der Zähne in einer Momentsituation durch die Anfertigung eines sogenannten Situationsabdruckes erfaßt. Nach dem Ausgießen des Situationsabdruckes mit Gipssuspension erhält der Zahnarzt dann das sogenannte Studienmodell, diagnostische Modell, Dokumentationsmodell, Arbeits- und Planungsmodell. Auch zur Darstellung des Gegenkiefers bei umfangreicheren prothetischen Arbeiten werden sogenannte Gegenkiefermodelle erstellt, die durch Abformungen mit Alginaten erhalten werden.

Ein wichtiger Einsatzbereich für Alginat-Abformmaterialien ist die Herstellung von provisorischen Kronen und Brücken. Hierbei wird an dem Patienten vor dem Beginn der Präparation eines oder mehrerer Zähne ein Situationsabdruck genommen, der die Ausgangssituation festhält. Nach erfolgter Präparation wird im Situationsabdruck an den entsprechenden Stellen ein provisorisches Kronen- und Brückenmaterial eingebracht, das zunächst noch im pastösen Zustand vorliegt. Dieser mit dem pastösen Material gefüllte Abdruck wird dann in den Mund des Patienten reponiert, wo das Kronen- und Brückenmaterial zum Provisorium aushärtet. Hier ist es von besonderer Bedeutung, daß das Alginatmaterial im ausgehärteten Zustand leicht beschneidbar ist, um die Formgebung des Provisoriums zugunsten der Stabilität durch entsprechendes Zuschneiden des Alginatabdrucks günstig zu beeinflussen.

Alginate bestehen aus Derivaten der Alginsäure, die nach Anrühren mit Wasser in einem Sol-Gel-Prozeß aushärten. Die Abformungen werden als sogenannte einphasige oder monophasige Abformungen vorgenommen, d.h. daß bei der Abdrucknahme nur eine einzige Konsistenz des Abformmaterials eingesetzt wird (siehe J. Wirz et al., "Abformung in der zahnärztlichen Praxis", 1993, Gustav Fischer Verlag, S. 7). Einige der wesentlichen Vorteile von Alginaten für die beschriebenen Einsatzbereiche sind:
- hohe Hydrophilie
- einphasige Materialien
- gutes Anfließen der Paste in Unterschnitten
- leichte Beschneidbarkeit des gehärteten Abdrucks
- leichte Entnehmbarkeit des gehärteten Abdrucks aus dem Mund
- geringe Kosten.

Weitere Vorteile der Alginatmaterialien sind
- gute Standfestigkeit, d.h. kein Ablaufen der Paste aus dem Abformlöffel
- kurze Abbindezeit, d.h. geringe Verweildauer der Abformmasse im Mund.

Der Einsatz von Alginaten ist jedoch auch mit einigen entscheidenden Nachteilen verbunden:
- keine Dimensions- und Lagerstabilität der gehärteten Abdrücke infolge des Austrocknens. Aufgrund dieses Wasserverlustes und dadurch bedingter Schrumpfung müssen die Alginatabdrücke sofort nach der Entnahme aus dem Mund mit Gipssuspension ausgegossen werden.
- kein mehrmaliges Ausgießen mit Gips möglich. Von jedem Abdruck kann nur ein Gipsmodell hergestellt werden. Zerbricht beispielsweise das Modell, ist eine erneute Abdrucknahme erforderlich.
- Desinfizierbarkeit ist problematisch aufgrund der Neigung zur Wasseraufnahme und zum Quellen.
- teilweise Gipsunverträglichkeit, so daß es zum Auftreten rauher Oberflächen auf dem Gipsmodell kommen kann.
- die Anmischung erfolgt in der Regel noch per Hand. Es existieren zwar einige automatische Mischsysteme, die aber nur von geringer Bedeutung sind und zudem auch kein automatisches Dosieren in den Löffel erlauben. Infolge der Handanmischung befinden sich häufig Luftblasen in den Abdrücken, was zu Ungenauigkeiten auf den Modellen führen kann.

Eine andere Klasse von dentalen Abformmaterialien stellen die additionsvernetzenden Silikone dar, die derzeit als Präzisionsabformmaterialien zur Erstellung von äußerst präzisen Arbeitsmodellen zur Herstellung von Zahnersatz verwendet werden. Die Eigenschaften derartiger Massen werden z.B. in den Normen ISO 4823 und ADA 19 beschrieben. Additionsvernetzende Silikone sind beispielsweise in der US-A-4 035 453 beschrieben. Da die Aushärtung bei ihnen nach einem platinkatalysierten Additionsmechanismus erfolgt, der nicht auf einer wäßrigen Basis funktioniert, weisen sie prinzipiell nicht das Phänomen der Dimensionsunstabilität infolge eines Wasserverlustes auf. Zudem sind Abdrücke aus additionsvernetzenden Silikonen beliebig oft mit Gipssuspension ausgießbar, so daß mit einem einzigen Abdruck mehrere Modelle mit glatter Gipsoberfläche gegossen werden können. Auch die Desinfizierbarkeit der Abdrücke ist bei additionsvernetzenden Silikonen unproblematisch.

Silikon-Abformmassen sind von Grund auf hydrophob und zeigen das Problem, daß die Zeichnungsschärfe des Abdrucks durch schlechtes Anfließverhalten der Paste infolge unzureichender Hydrophilie nicht befriedigend ist. Diese Problematik ist z.B. in der DE-A-38 38 587, Seite 2, Zeilen 19-23 oder in der EP-A-0 480 238, Seite 2, Zeilen 1-26 dargestellt.

In der Literatur sind zur Lösung dieses Problems verschiedene Zusätze beschrieben worden, die die Hydrophilie von Silikon-Abdruckmassen erhöhen. Ein Überblick über den Stand der Technik findet sich beispielsweise in EP-A-0 480 238, Seite 2, Zeilen 20-38. Als besonders wirksame Zusätze haben sich Polyethersiloxane erwiesen, wie sie beispielsweise in der internationalen Anmeldung WO 87/03001 oder in der EP-B-0 231 420 beschrieben werden. Weitere sehr wirksame Zusätze sind sogenannte Polyethercarbosilane, wie sie in WO 96/08230 dargestellt sind. Auch ethoxylierte Fettalkoholderivate, siehe z.B. EP-B-0 480 238, sind zur Erhöhung der Hydrophilie und damit zur Verbesserung des Anfließverhaltens geeignet. Durch den Einsatz dieser Tenside wird das Anfließverhalten der Pasten erheblich verbessert, so daß eine gute Benetzbarkeit erreichbar ist.

Handelsübliche additionsvernetzende Silikonabformmassen liegen üblicherweise in einer zweikomponentigen Form vor und bestehen aus einer sogenannten Basis- und Katalysatorpaste, in der die reaktiven Komponenten aus Stabilitätsgründen räumlich voneinander getrennt sind. Die Aushärtung der Materialien erfolgt nach dem Anmischen der beiden Pasten in genau definierten Volumenverhältnissen. Die Anmischung erfolgt in der Regel manuell oder durch das Ausdrücken aus Doppelkammerkartuschen, wobei die Pasten durch ein Mischrohr gefördert werden, das einen statischen Mischer enthält, wodurch es zu einer innigen Vermischung der Pasten kommt. Damit sind aber nur relativ kleine Pastenmengen in kurzer Zeit anmischbar.

Eine Fortentwicklung der Handlingeigenschaften additionsvernetzender Silikonabformmassen besteht in der Entwicklung automatischer Anmisch- und Dosiersysteme für zweikomponentige Abformmassen, die über automatische Förder- und Mischeinheiten verfügen, wie sie z.B. in der US-A-5 249 862, US-A-5 286 105 und US-A-5 332 122 beschrieben sind. Damit wird die Notwendigkeit eines manuellen Mischens von Basis- und Katalysatorpasten vor allem bei der Anmischung größerer Materialmengen hinfällig, da dies automatisch und in kurzer Zeit erfolgen kann. Das Resultat ist ein völlig homogenes Produkt, das frei von Blasen ist (siehe Prospekt ESPE Pentamix®, Fa. ESPE, Seefeld).

Additionsvernetzende Silikone werden vorwiegend zweiphasig angewendet: Leichtfließende oder mittelfließende Massen werden mit schwerfließenden oder Knetmassen kombiniert (siehe J. Wirz et al., S. 7).

Es gibt zwar hydrophile additionsvernetzende Silikone, die in einer Monophasentechnik eingesetzt werden, d.h. in nur einer einzigen Konsistenz zur Anwendung kommen. An den Einsatz derartiger Silikonabformmassen für die Herstellung von provisorischen Brücken und Kronen hat man bislang nicht gedacht, da diese Materialien für die Präzisionsabformung eingesetzt werden, was sich vom Einsatz zur Situationsabformung grundlegend unterscheidet. So haben handelsübliche hydrophile Monophasen-A-Silikone eine recht langsame Abbindung, die in der Regel bei einer Abbindezeit im Mund (siehe J. Wirz, "Abformung in der zahnärztlichen Praxis", 1993, Gustav Fischer Verlag, S. 16 bis 26) von über 3 Minuten liegt. Die Abbindezeit im Mund ist die Zeit zwischen Positionierung des Löffels mit dem Abformmaterial im Mund des Patienten und der Entnahme des ausgehärteten Abdrucks und kann auch als Mundverweildauer oder -zeit bezeichnet werden. Dies ist für ein Situationsabformmaterial ungünstig, da der Zahnarzt bei dieser Art der Abformung möglichst zeitsparend arbeiten will. Abbindezeiten von < 3 Minuten Mundverweildauer, bevorzugt < 2,5 Minuten und besonders bevorzugt < 2 Minuten, sind für den Zahnarzt hierbei gewünschte Eigenschaften. Beispielsweise weist das hydrophile Einphasenabformmaterial "Imprint 2:5" (Fa. 3M) eine Abbindezeit von 5 Minuten auf, wohingegen ein typisches Alginatabformmaterial ("Alginoplast", Fa. Bayer) eine Abbindezeit von 1,5 Minuten zeigt.

Zudem sind hydrophile Monophasen-A-Silikone teure Abformmassen und weisen in der Regel im ausgehärteten Zustand recht hohe Härten auf, so daß ihre Shore-Härte A - gemessen nach DIN 53505, 30 Minuten nach dem Anmischbeginn der Pasten - in der Regel > 50 beträgt. Dadurch lassen sich solche Abformmassen wegen ihrer harten Beschaffenheit nur relativ schlecht von den abgeformten Gegenständen trennen.

Ebenso läßt sich das im Mund des Patienten zum Provisorium ausgehärtete Kronen- und Brückenmaterial schlecht vom Abformmaterial trennen, wenn dieses eine zu hohe Härte besitzt. Man könnte zwar daran denken, die bekannten hydrophilen Monophasen-Silikonabformmaterialien durch Zusätze so zu modifizieren, daß sie im ausgehärteten Zustand weicher und flexibler sind, so daß sie dann leicht von den abgeformten Gegenständen getrennt werden können. Ein Zusatz von Weichmachern, wie z.B. handelsübliche Weichmacher für Kunststoffe oder Silikonöle, führt auch zur Reduktion der Kosten. Derartig weichgemachte Silikonabformmaterialien sind dann aber in ihrer Beschneidbarkeit nicht akzeptabel. Bei der Herstellung der provisorischen Kronen und Brücken muß der Zahnarzt den gehärteten Abdruck an den interdentalen Stellen beschneiden, damit das von Haus aus relativ spröde Material, das für die Herstellung der Provisorien verwendet wird, nicht an den interdentalen Stellen zu dünn wird und bricht. Versuche mit lediglich weichgemachten hydrophilen Monophasen-Silikonabformmaterialien, die nach dem Aushärten eine Shore-Härte A von weniger als 50 aufwiesen, waren nicht mehr in der notwendigen Weise beschneidbar, da die Interdentalsepten in der üblichen Schnittechnik einem Skalpell elastisch ausweichen.

Die Aufgabe der Erfindung besteht darin, ein für die Herstellung von provisorischen Brücken und Kronen geeignetes Abformmaterial zur Verfügung zu stellen, das zwar die Vorteile, nicht aber die Nachteile von Alginatabformmaterialien aufweist.

Insbesondere sollen die Massen für eine automatische Anmischung im Pentamix-Mischgerät geeignet sein. Dies bedeutet, daß ein Mischungsverhältnis in einer zweikomponentigen Darreichungsform von 5:1 einstellbar sein muß. Die Pasten müssen dabei eine ausreichende Standfestigkeit im angemischten Zustand aufweisen, damit sie nach dem Einbringen nicht aus dem Abformlöffel in den Mund des Patienten abtropfen. Dennoch muß aber eine ausreichende Fließfähigkeit gegeben sein, so daß ein Fördern und Anmischen im Pentamix-Gerät in geeigneter Weise möglich ist.

Überraschenderweise wurde im Rahmen der vorliegenden Erfindung festgestellt, daß diese Aufgabe mit in besonderer Weise modifizierten Silikonabformmaterialien gelöst werden kann.

Die Erfindung stellt somit ein hydrophiles, monophasisches additionsvernetzendes Silikonabformmaterial zur Verfügung, welches die folgenden Bestandteile enthält:
(a) Organopolysiloxane mit mindestens zwei ungesättigten Gruppen im Molekül,
(b) Organohydrogenpolysiloxane mit mindestens 2 SiH-Gruppen im Molekül,
(c) gegebenenfalls Organopolysiloxane ohne reaktive Gruppen,
(d) Platinkatalysator,
(e) Hydrophilierungsmittel,
(f) Diatomeenerde,
(g) Füllstoff, sowie
(h) gegebenenfalls weitere übliche Zusatz-, Hilfs- und Farbstoffe,
wobei die Menge an Komponente (f) 8 bis 25 Gew.-%, bevorzugt 10 bis 20 %, bezogen auf die Gesamtmasse des ausgehärteten Gummis beträgt, und die Masse eine nach DIN 53505 zu bestimmende Shore-Härte A von kleiner als 45 - gemessen 30 Minuten nach Anmischung der Pasten - bevorzugt < 40, und eine nach ISO 4823 zu bestimmende Konsistenz von 31 bis 39 mm aufweist.

Die Massen verfügen über eine Abbindezeit im Mund von < 2,5 Minuten, bevorzugt < 2 Minuten, besonders bevorzugt ≤ 1,5 Minuten. Die Materialien verfügen über eine hohe Hydrophilie. Die Hydrophilie kann bestimmt werden durch die Messung des Randwinkels eines Wassertropfens oder eines Tropfens aus gesättigter Calciumsulfatlösung zur Oberfläche einer ausgehärteten Probe des Abformmaterials. Die Meßmethodik ist beispielsweise in der DE-A-43 06 997, Seite 5, beschrieben. Der 3-Minuten-Wert des Kontaktwinkels beträgt bevorzugt < 60°, besonders bevorzugt < 50°, insbesondere < 40°.

Mit einer Shore-Härte A von kleiner als 45 ist die Entnehmbarkeit des gehärteten Abdruckes aus dem Mund aufgrund der Weichheit des Gummis als sehr leicht zu bezeichnen. Überraschenderweise wird durch die Anwesenheit von Komponente (f) in den angegebenen Mengenbereichen trotz der Weichheit des Materials eine sehr gute Beschneidbarkeit erzielt, so daß die Silikonabformmasse als Alginatsubstitut herangezogen werden kann.

Die prinzipielle Möglichkeit der Verwendung von Diatomeenerde in hydrophilen Silikonen ist zwar bekannt und wird beispielsweise in EP-B-0 480 238, Seite 3, Zeile 33, sowie in WO 93/04659, Seite 12, beschrieben. Es findet sich in diesen Druckschriften aber kein Hinweis auf den positiven Einfluß auf die Beschneidbarkeit von Silikonen mit einer geringen Shore-Härte durch den Zusatz von Diatomeenerde innerhalb der beschriebenen Mengenbereiche.

Bevorzugt sind als Komponente (a) Diorganopolysiloxane mit terminalen Triorganosiloxygruppen, von denen mindestens eine der drei organischen Gruppen eine Vinylgruppe ist. Bevorzugte Diorganosiloxane dieser Struktur werden durch folgende Formel wiedergegeben: 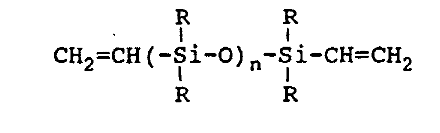 in der R eine unsubstituierte oder substituierte monovalente Kohlenwasserstoffgruppe repräsentiert, die bevorzugt frei von aliphatischen Mehrfachbindungen ist und n eine ganze Zahl darstellt. Mindestens 50 % der Reste R sind bevorzugt Methylgruppen und Beispiele für andere Gruppen R sind Ethyl-, Vinyl- und 3,3,3-Trifluorpropylgruppen. Der Wert von n soll so liegen, daß das Polymer bei 25°C eine Viskosität zwischen 200 bis 200.000 mPa·s, bevorzugt 1000 bis 10.000 mPa·s aufweist. Derartige Moleküle sind in der US-A-4 035 453 beschrieben. Die Herstellung der Komponenten (a) erfolgt nach üblichen Verfahren, die z.B. in W. Noll, "Chemie und Technologie der Silikone", Verlag Chemie Weinheim, 2. Auflage 1964, Seite 162-206 oder J. Burghardt, Chemie und Technologie der Polysiloxane in "Silikone, Chemie und Technologie", Vulkan Verlag, Essen, 1989, Seiten 23-37 dargestellt sind.

Besonders bevorzugt sind lineare Polydimethylsiloxane obiger Struktur mit den angegebenen Viskositätsbereichen, bei denen die Endgruppen aus Dimethylvinylsiloxyeinheiten bestehen und die weiteren Substituenten R in der Kette aus Methylgruppen bestehen.

Komponente (b) ist bevorzugt ein Organopolysiloxan mit mindestens 3 Si-gebundenen Wasserstoffatomen pro Molekül. Dieses Organopolysiloxan enthält bevorzugt von 0,01 bis 1,7 Gew.-% siliciumgebundenen Wasserstoff. Die Silikonvalenzen, die nicht mit Wasserstoff- oder Sauerstoffatomen abgesättigt sind, werden mit monovalenten Kohlenwasserstoffradikalen abgesättigt, die frei von aliphatischen Mehrfachbindungen sind. Die Kohlenwasserstoffradikale können substituiert oder unsubstituiert sein. Mindestens 50 %, bevorzugt 100 %, der Kohlenwasserstoffradikale, die an Siliciumatome gebunden sind, bestehen aus Methylradikalen. Auch derartige Komponenten sind in den oben genannten Literaturstellen hinsichtlich Struktur und Herstellung beschrieben.

Die Mengenverhältnisse der Komponenten (a) und (b) sind bevorzugt derart gewählt, daß pro Mol an ungesättigter Doppelbindung der Komponente (a) 0,75 bis 5 Mol SiH-Einheiten aus der Komponente (b) vorliegen. Die Summe der Komponenten (a) und der Komponente (b) liegt im Bereich von 10 bis 40 Gew.-%, bezogen auf das Gesamtgewicht aller Komponenten. Bevorzugt liegen sie im Bereich von 15 bis 25 Gew.-%.

Geeignete Komponenten (c) sind polymere Organosiloxane ohne reaktive Substituenten, wie sie z.B. in W. Noll "Chemie und Technologie der Silikone", Verlag Chemie Weinheim, 1968, Seite 212 ff. beschrieben sind. Es handelt sich bevorzugt um lineare, verzweigte oder cyclische Organopolysiloxane, bei denen sämtliche Siliciumatome von Sauerstoffatomen oder monovalenten Kohlenwasserstoffradikalen umgeben sind, wobei die Kohlenwasserstoffradikale substituiert oder unsubstituiert sein können. Die Kohlenwasserstoffradikale können Methyl, Ethyl, C₂-C₁₀-Aliphaten, Trifluorpropylgruppen, sowie aromatische C₆-C₁₂-Substituenten sein. Die Komponente (c) trägt zur Verdünnung und Aufweitung des Gumminetzwerkes bei und wirkt als Weichmacher für das ausgehärtete Material. Als relativ günstige Komponente trägt sie auch bei zur Reduktion der Herstellungskosten der erfindungsgemäßen Abformmassen.

Besonders bevorzugt als Komponente (c) sind Polydimethylsiloxane, welche Trimethylsiloxy-Endgruppen aufweisen. Die Viskosität der Komponente (c) liegt bevorzugt im Bereich von 40 bis 2000 mPa·s, besonders bevorzugt 50 bis 1000 mPa·s. Die Menge an Komponente (c) beträgt 0 bis 40 Gew.-%, bevorzugt 5 bis 40 Gew.-%, besonders bevorzugt 15 bis 30 Gew.-%.

Komponente (d) ist bevorzugt ein Platinkomplex, der aus Hexachlorplatinsäure durch Reduktion mit Tetramethyldivinyldisiloxan hergestellt wurde. Diese Verbindungen sind an sich bekannt. Geeignet sind auch andere Platinverbindungen, die die Additionsvernetzung beschleunigen. Gut geeignet sind z.B. Platin-Siloxan-Komplexe, wie sie z.B. in den US-A-3 715 334, US-A-3 775 352 und US-A-3 814 730 beschrieben sind. Der Platinkatalysator wird vorzugsweise in Mengen von 0,00005 bis 0,05 Gew.-%, insbesondere 0,0002 bis 0,04 Gew.-%, jeweils.berechnet als elementares Platin und bezogen auf das Gesamtgewicht der mit den Komponenten (a) bis (h) vorliegenden Masse, eingesetzt. Zur Steuerung der Reaktivität kann es notwendig sein, daß ein Inhibitor zugesetzt werden muß, der die vorzeitige Vernetzung zum Elastomeren behindert. Derartige Inhibitoren sind bekannt und z.B. in US-A-3 933 880 beschrieben. Beispiele hierfür sind acetylenisch ungesättigte Alkohole, wie 3-Methyl-1-butin-3-ol, 1-Ethinylcyclohexan-1-ol, 3,5-Dimethyl-1-hexin-3-ol und 3-Methyl-1-pentin-3-ol. Beispiele für Inhibitoren auf Vinylsiloxanbasis sind 1,1,3,3-Tetramethyl-1,3-divinylsiloxan und vinylgruppenhaltige Poly-, Oligo- und Disiloxane.

Komponente (e) ist ein eine hydrophile Natur verleihendes Mittel oder Hydrophilierungsmittel, das den Randwinkel eines Tropfens Wasser oder wäßriger Zusammensetzung (z.B. Gipssuspension, etc.) gegenüber der Silikonzusammensetzung erniedrigt und damit eine bessere Benetzbarkeit der Gesamtzusammensetzung im feuchten Mundmilieu und damit ein besseres Anfließverhalten der Pasten hervorruft. Die Messung des Randwinkels zur Bestimmung der Hydrophilie der Abformmassen ist z.B. in der DE-A-43 06 997, Seite 5, beschrieben. Die Hydrophilierungsmittel sind bevorzugt nicht mit reaktiven Gruppen versehen, so daß kein Einbau in das Polysiloxannetzwerk stattfindet. Geeignete Hydrophilierungsmittel sind bevorzugt nicht einbaubare Benetzungsmittel aus der Gruppe der hydrophilen Silikonöle, die in WO 87/03001 und in EP-B-0 231 420 beschrieben sind. Bevorzugt sind ferner die ethoxylierten Fettalkohole, die in EP-B-0 480 238 beschrieben sind. Desweiteren sind bevorzugte Hydrophilierungsmittel die aus WO 96/08230 bekannten Polyethercarbosilane. Bevorzugt sind auch die in WO 87/03001 beschriebenen nicht-ionischen perfluoralkylierten oberflächenaktiven Substanzen. Ebenfalls bevorzugt sind die nicht-ionischen oberflächenaktiven Substanzen, die in EP-B-0 268 347 beschrieben sind, d.h. die darin aufgeführten Nonylphenolethoxylate, Polyethylenglykol-monound -diester, Sorbitanester, sowie Polyethylenglykol-mono- und -diether. Die Einsatzmengen der Hydrophilierungsmittel betragen 0,1 bis 10 Gew.-%, bezogen auf das Gesamtgewicht aller Komponenten, bevorzugt 0,2 bis 2 Gew.-% und besonders bevorzugt 0,3 bis 1 Gew.-%. Der nach 3 Min. gemessene Randwinkel eines Wassertropfens auf der Oberfläche eines ausgehärteten erfindungsgemäßen Materials beträgt bevorzugt weniger als 60°, besonders bevorzugt < 50°, insbesondere < 40°.

Komponente (f) wird als Diatomeenerde oder Kieselgur bezeichnet. Sie besteht aus den sehr mannigfaltig geformten Kieselsäuregerüsten einzelliger, mikroskopisch kleiner, in Süß- oder Salzwasser lebender Algen (Diatomeen). Die Materialien werden meist im Tagebau gefördert und auch als Infusorienerde, Bergmehl oder Bacillenerde bezeichnet. Bevorzugt eingesetzte Typen von Diatomeenerde werden in calcinierter Form eingesetzt. Hierbei wird beispielsweise ein Trocknen in Drehrohröfen und anschließendes Erhitzen auf ca. 700°C vorgenommen, wobei organische Bestandteile verbrennen. Hierbei können auch eventuell NaCl oder Ammoniumchlorid zugesetzt werden, wobei unerwünschtes Eisen in flüchtiges Eisentrichlorid verwandelt wird. Das Rohmaterial kann auch nach anderen besonderen Verfahren aufgebracht werden, z.B. mit Flußmitteln, wie Na₂CO₃, KOH oder NaOH. Komponente (f) kann auch oberflächenmodifiziert, beispielsweise silanisiert, vorliegen. Geeignete Verfahren und Mittel sind bei der Beschreibung der Komponente (g) beschrieben. Die Einsatzmengen betragen 8 bis 25, vorzugsweise 10 bis 20 Gew.-%, bezogen auf die Gesamtmenge der Komponenten. Bei einer darunter liegenden Einsatzmenge wird bei erfindungsgemäßen ausgehärteten Elastomeren im angegebenen Shore-Härte-Bereich von < 45 (gemessen nach 30 Minuten nach Anmischung der Paste),die Beschneidbarkeit nicht in ausreichendem Maße verbessert. Bei Einsatzmengen, die darüber liegen, kommt es infolge von Entmischungseffekten zu Instabilitäten der Pasten im Laufe der Lagerung. Bevorzugte Typen von Diatomeenerde sind z.B. die Produkte mit dem Handelsnamen "Celatom" (Vertrieb durch z.B. Fa. Chemag), "Cellite 219", "Cellite 499", "Cellite 263 LD", "Cellite 281" und "Cellite 281 SS" der Fa. Johns-Manville, sowie "Diatomite 104", "Diatomite CA-3", "Diatomite IG-33", "Diatomite 143", "Diatomite SA-3", "Diatomite 183" der Fa. Dicallite, ebenso wie die Produkte "Clarcel" der Fa. Ceca.

Zu den einsetzbaren Füllstoffen gemäß Komponente (g) gehören nicht verstärkende Füllstoffe mit einer BET-Oberfläche von bis zu 50 m²/g, wie Quarz, Christobalit, Calciumsilikat, Zirkoniumsilikat, Montmorillonite wie Benthonite, Zheolite, einschließlich der Molekularsiebe, wie Natriumaluminiumsilikat, Metalloxidpulver, wie Aluminium- oder Zinkoxide bzw. deren Mischoxide, Bariumsulfat, Calciumcarbonat, Gips, Glas- und Kunststoffpulver. Zu möglichen Füllstoffen gehören auch verstärkende Füllstoffe mit einer BET-Oberfläche von mehr als 50 m²/g, wie z.B. pyrogene oder gefällte Kieselsäure und Siliciumaluminiummischoxide mit großer BET-Oberfläche. Die genannten Füllstoffe können hydrophobiert sein, beispielsweise durch die Behandlung mit Organosilanen bzw. -siloxanen oder durch die Veretherung von Hydroxylgruppen zu Alkoxygruppen. Es kann eine Art von Füllstoff, es kann auch ein Gemisch von mindestens zwei Füllstoffen eingesetzt werden. Die Kornverteilung wird vorzugsweise so gewählt, daß keine Füllstoffe mit Korngrößen > 50 µm enthalten sind. Der Gesamtgehalt der Füllstoffe (g) liegt im Bereich von 10 bis 80 %, bevorzugt 30 bis 60 %, wobei die Füllstoffmengen so gewählt werden, daß eine Shore-Härte A des ausgehärteten Gummis von < 45 nach 30 Minuten nicht überschritten wird.

Besonders bevorzugt ist eine Kombination aus verstärkenden und nicht verstärkenden Füllstoffen. Hierbei liegen die verstärkenden Füllstoffe in Mengenbereichen von 1 bis 10 Gew.-%, insbesondere 2 bis 5 Gew.-%. Die Differenz zu den genannten Gesamtbereichen, also 9 bis 70 Gew.-%, insbesondere 28 bis 55 Gew.-%, bilden die nicht verstärkenden Füllstoffe.

Bevorzugt als verstärkende Füllstoffe sind pyrogen hergestellte hochdisperse Kieselsäuren, die bevorzugt durch Oberflächenbehandlung hydrophobiert worden sind. Die Oberflächenbehandlung kann beispielsweise mit Dimethyldichlorsilan, Hexamethyldisilasan, Tetramethylcyclotetrasiloxan oder Polymethylsiloxanen erfolgen. Die Oberflächen geeigneter pyrogener Kieselsäuren betragen bevorzugt > 50 m²/g, insbesondere 80 bis 150 m²/g. Die Anwesenheit der oberflächenbehandelten pyrogenen Kieselsäuren trägt zur Einstellung der Konsistenz und zur Verbesserung der Standfestigkeit der Pasten bei. Bei Mengen von < 1 Gew.-% ist in der Regel kein merkbarer Einfluß auf die Standfestigkeit feststellbar, Mengen von > 10 Gew.-% führen in der Regel zu einer zu starken Verdickung der Pasten, so daß keine ausreichende Fließfähigkeit mehr erhalten werden kann. Dies führt bei der automatischen Anmischung im Pentamix-Gerät zu unerwünschten Temperaturerhöhungen, die zu einer zu raschen Pastenaushärtung führen können. Geeignete Produkte sind beispielsweise in den Prospekten der Fa. Degussa (Aerosil-Produkte, Schriftenreihe Pigmente, Nr. 11, 5. Auflage, 1991, auf Seite 79) sowie der Fa. Cabot Corp. (Cabosil-Produkte, "CAB-O-SIL Fumed silica in Adhesives and Sealants, Cabot, 1990) beschrieben.

Besonders bevorzugte nichtverstärkende Füllstoffe sind Quarze, Christobalite und Natriumaluminiumsilikate, die oberflächenbehandelt sein können. Die Oberflächenbehandlung kann prinzipiell mit den gleichen Methoden erfolgen wie im Fall der verstärkenden Füllstoffe beschrieben.

Weiterhin können die erfindungsgemäßen Abformmassen als Komponente (h) gegebenenfalls Zusätze wie Weichmacher, Pigmente, Antioxidationsmittel, Trennmittel, u.ä. enthalten. Ebenso können auch beispielsweise fein verteiltes Palladium oder Platin als Wasserstoffabsorber enthalten sein. Die Metalle können dabei auch auf Trägermaterialien aufgebracht sein. Die erfindungsgemäßen Massen enthalten derlei Zusatzstoffe in Mengen von vorzugsweise 0 bis 2 Gew.-%, besonders bevorzugt von 0,1 bis 1 Gew.-%.

Die Massen werden hergestellt durch Vermischen der Komponenten (a) bis (h) und härten in einer als Hydrosilylierung bezeichneten Additionsreaktion aus, bei der sich unter dem Einfluß des Platinkatalysators (d) die SiH-Gruppen der Komponente (b) an die ungesättigten Gruppen der Komponente (a) addieren. Aus Lagerstabilitätsgründen ist es bevorzugt, die Massen in einer zweikomponentigen Darreichungsform zu formulieren, bei der die gesamte Komponente (b) in einer sogenannten Basispaste untergebracht ist. Räumlich getrennt hiervon wird die gesamte Komponente (d) in einer sogenannten Katalysatorpaste untergebracht. Die Komponente (a) kann entweder in der Katalysator- oder Basispaste untergebracht sein, wobei bevorzugt je ein Teil der Komponente (a) in der Basis- und ein Teil der Komponente (a) in der Katalysatorpaste untergebracht ist. Die Komponenten (c), (e), (f), (g) und (h) können in ihrer Gesamtmenge in der Katalysator- oder in der Basispaste untergebracht sein, wobei bevorzugt ist, daß jeweils ein Teil der jeweiligen Komponente in der Katalysator- und ein Teil in der Basispaste untergebracht ist. Besonders bevorzugt ist, daß die Komponenten (e) und (f) nur in der Basispaste enthalten sind.

Die Volumenverhältnisse von Katalysator- und Basispaste können 10:1 bis 1:10 betragen. Besonders bevorzugte Volumenverhältnisse von Basis- : Katalysatorpaste sind 1:1 und 5:1 (5 Teile Basis- : 1 Teil Katalysatorpaste). Im Falle eines Volumenverhältnisses von 1:1 können die Komponenten (a) bis (h) wie folgt auf Basis- und Katalysatorpaste verteilt sein.

**Tabelle 1**

| Komponente | Basispaste | Katalysatorpaste | Summe in Basis und Katalysator |
|---|---|---|---|
| (a) | 10-48 % | 10-48 % | 14-24 % |
| (b) | 2-10 % | - | 1-5 % |
| (c) | 0-60 % | 0-60 % | 15-30 % |
| (d) | - | 0,0004 - 0,08 % Pt | 0,0002 - 0,04 % Pt |
| (e) | 0-4 % | 0-4 % | 0,2-2 % |
| (f) | 0-40 % | 0-40 % | 10-20 % |
| (g) verstärkende Füllstoffe | 0-10 % | 0-10 % | 2-5 % |
| nicht verstärkende Füllstoffe | 10-55 % | 10-55 % | 28-55 % |
| (h) | 0-1 % | 0-1 % | 0,1-1 % |

Im Falle eines Volumenverhältnisses von 5 Teilen Basispaste zu 1 Teil Katalysatorpaste können bevorzugte Mengenanteile wie folgt wiedergegeben werden:

**Tabelle 2**

| Komponente | Basispaste | Katalysatorpaste | Summe in Basis und Katalysator |
|---|---|---|---|
| (a) | 2-29 % | 0-50 % | 14-24 % |
| (b) | 1-6 % | - | 1-5 % |
| (c) | 0-36 % | 0-30 % | 15-30 % |
| (d) | - | 0,002 - 0,2 % Pt | 0,0002 - 0,04 % Pt |
| (e) | 0-2,4 % | 0-12 % | 0,2-2 % |
| (f) | 0-24 % | 0-70 % | 10-18 % |
| (g) verstärkende Füllstoffe | 0-6 % | 0-30 % | 2-5 % |
| nicht verstärkende Füllstoffe | 10-60 % | 10-70 % | 28-55 % |
| (h) | 0-1,2 % | 0-1 % | 0,1-1 % |

Bei einem Volumenverhältnis 5:1 können die beiden Pasten in Schlauchfolienbeutel abgefüllt und später kurz vor der Verwendung mit Hilfe des Misch- und Dosiergerätes PENTAMIX® (Fa. ESPE) angemischt werden.

### Beispiele

### Referenzbeispiel 1

44,3 Teile eines Vinyl-endgestoppten Polydimethylsiloxans mit einer Viskosität von 2000 mPa·s bei 23°C, 4,6 Teile eines SiH-Gruppen-haltigen Polydimethylsiloxans mit einer Viskosität von 60 mPa·s bei 23°C, 3,9 Teile eines Polydimethylsiloxans mit einer Viskosität von 50 mPa·s bei 23°C, 5,8 Teile einer silanisierten pyrogenen Kieselsäure, 40,3 Teile silanisiertes Quarzfeinstmehl und 1,1 Teile des Carbosilantensid-Hydrophilierungsmittels gemäß WO 96/08230, Herstellungsbeispiel 2, werden in einem Kneter durch Mischen zu einer homogenen Basispaste vereinigt.

Die Katalysatorpaste wird durch Vermischen von 42,0 Teilen eines Vinyl-endgestoppten Polydimethylsiloxans mit einer Viskosität von 2000 mPa·s bei 23°C, 1,5 Teilen einer silanisierten pyrogenen Kieselsäure, 49,0 Teilen Natriumaluminiumsilikatfüllstoff, 7,0 Teilen einer Lösung eines Komplexes aus Platin und Divinyltetramethyldisiloxan, enthaltend 1,3 Gew.-% Platin in einem Vinyl-endgestoppten Polydimethylsiloxans mit einer Viskosität von 2000 mPa·s bei 23°C und 0,5 Teilen Farbpigment hergestellt.

50 g Basispaste und 10 g Katalysatorpaste werden vollständig vermischt. Nach einigen Minuten erhält man eine gummielastische Masse. 30 Minuten nach Herstellung wird die Shore-Härte A zu 38 bestimmt. Die Konsistenz der angemischten Pasten wird gemäß ISO 4823 zu 37 mm bestimmt. 30 Minuten nach Herstellung wird der Randwinkel nach 3 Minuten Benetzungszeit mit 20° bestimmt.

Der ausgehärtete Gummi enthält keine Diatomeenerde und ist mit dem Skalpell schlecht beschneidbar. Die Abbindezeit im Mund beträgt 3 Minuten 15 Sekunden. Lagerstabilität: Nach 4 Wochen Einlagerung sind die Pasten unverändert.

### Beispiel 1

17,1 Teile eines Vinyl-endgestoppten Polydimethylsiloxans mit einer Viskosität von 7000 mPa·s bei 23°C, 2,7 Teile eines SiH-Gruppen-haltigen Polydimethylsiloxans mit einer Viskosität von 60 mPa·s bei 23°C, 25,3 Teile eines Polydimethylsiloxans mit einer Viskosität von 50 mPa·s bei 23°C, 4,0 Teile einer silanisierten pyrogenen Kieselsäure, 35,3 Teile silanisiertes Quarzfeinstmehl, 0,4 Teile Farbpigment und 14,7 Teile Diatomeenerde (1 bis 20 µm) und 0,5 Teile des Carbosilantensid-Hydrophilierungsmittels gemäß WO 96/08230, Herstellungsbeispiel 2, werden in einem Kneter durch Mischen zu einer homogenen Basispaste vereinigt.

Die Katalysatorpaste wird durch Vermischen von 19,9 Teilen eines Vinyl-endgestoppten Polydimethylsiloxans mit einer Viskosität von 7000 mPa·s bei 23°C, 7,7 Teile eines Polydimethylsiloxans mit einer Viskosität von 50 mPa·s bei 23°C, 1,8 Teile einer silanisierten pyrogenen Kieselsäure, 66,3 Teile Natriumaluminiumsilikatfüllstoff, 0,02 Teile Farbpigment, 4,3 Teilen einer Lösung eines Komplexes aus Platin und Divinyltetramethyldisiloxan, enthaltend 1,3 Gew.-% Platin in Vinyl-endgestopptem Polydimethylsiloxan mit einer Viskosität von 2000 mPa·s bei 23°C hergestellt.

50 g Basispaste und 10 g Katalysatorpaste werden vollständig vermischt. Nach einigen Minuten erhält man eine gummielastische Masse. Die Konsistenz gemäß ISO 4823 wird zu 34 mm bestimmt. Die Shore-Härte A nach 30 Minuten beträgt 35. 30 Minuten nach Herstellung wird der Randwinkel nach 3 Minuten Benetzungszeit mit 26° bestimmt.

Der ausgehärtete Gummi ist mit dem Skalpell sehr gut beschneidbar. Die Abbindezeit im Mund beträgt 1,5 Minuten. Lagerstabilität: Nach 4 Wochen Einlagerung sind die Pasten unverändert.

### Vergleichsbeispiel 1

39,8 Teile eines Vinyl-endgestoppten Polydimethylsiloxans mit einer Viskosität von 7000 mPa·s bei 23°C, 11,9 Teile eines SiH-Gruppen-haltigen Polydimethylsiloxans mit einem ungefähren Verhältnis von Dimethylsiloxy- : Methylhydrogensiloxygruppen von 10:1, 3,0 Teile calciniertes Kieselgur, 5,0 Teile silanisierte pyrogene Kieselsäure, 33,8 Teile silanisierter Quarz, 1,5 Teile Farbpigment und 5,0 Teile eines Carbosilantensid-Hydrophilierungsmittels gemäß WO 96/08230, werden in einem Kneter durch Mischen zu einer homogenen Basispaste vereinigt.

Die Katalysatorpaste wird durch Vermischen von 50,6 Teilen eines Vinyl-endgestoppten Polydimethylsiloxans mit einer Viskosität von 7000 mPa·s bei 23°C, 1,2 Teilen einer Lösung eines Komplexes aus Platin und Divinyltetramethyldisiloxan mit einem Platingehalt von 1,3 %, 3,6 Teilen calciniertes Kieselgur, 2,0 Teilen silanisierter pyrogener Kieselsäure, 41,7 Teilen eines silanisierten Quarzpulvers, 0,01 Teilen eines Farbpigments mit 1,0 Teilen eines Carbosilantensid-Hydrophilierungsmittels gemäß WO 96/08230, Herstellungsbeispiel 2, hergestellt.

10 g Basispaste und 10 g Katalysatorpaste werden vollständig vermischt. Nach einigen Minuten erhält man eine gummielastische Masse. Die Konsistenz gemäß ISO 4823 wird zu 36 mm bestimmt. Die Shore-Härte A beträgt 30 Minuten nach Herstellung des Prüfkörpers 36. 30 Minuten nach Herstellung wird der Randwinkel nach 3 Minuten Benetzungsdauer zu 20° bestimmt.

Der ausgehärtete Gummi ist mit dem Skalpell schlecht beschneidbar. Die Abformmasse dieses Vergleichsbeispiels enthält zwar Diatomeenerde, jedoch in einer zu geringen Menge. Lagerstabilität: Nach 4 Wochen Einlagerung sind die Pasten unverändert.

### Vergleichsbeispiel 2

19,4 Teile eines Vinyl-endgestoppten Polydimethylsiloxans mit einer Viskosität von 7000 mPa·s bei 23°C, 3,1 Teile eines SiH-Gruppen-haltigen Polydimethylsiloxans mit einer Viskosität von 60 mPa·s bei 23°C, 32,2 Teile eines Polydimethylsiloxans mit einer Viskosität von 50 mPa·s bei 23°C, 0,3 Teile einer silanisierten pyrogenen Kieselsäure, 44 Teile calciniertes Kieselgur (1 bis 20 µm), 0,3 Teile anorganisches Farbpigment dispergiert in0,2 Teilen eines Polydimethylsiloxans mit einer Viskosität von 50 mPa·s bei 23°C, und 0,6 Teile eines Carbosilantensid-Hydrophilisierungsmittels gemäß WO 96/08230, Herstellungsbeispiel 2, werden in einem Kneter durch Mischen zu einer homogenen Basispaste vereinigt.

Die Katalysatorpaste wird durch Vermischen von 42,0 Teilen eines Vinyl-endgestoppten Polydimethylsiloxans mit einer Viskosität von 7000 mPa·s bei 23°C, 1,5 Teilen einer silanisierten pyrogenen Kieselsäure, 49,0 Teilen Natriumaluminiumsilikat, 7,0 Teilen einer Lösung eines Komplexes aus Platin und Divinyltetramethyldisiloxan, enthaltend 1,3 Gew.-% Platin in Vinyl-endgestopptem Polydimethylsiloxan mit einer Viskosität von 2000 mPa·s bei 23°C und 0,5 Teilen Farbpigment hergestellt.

50 g Basispaste und 10 g Katalysatorpaste werden vollständig vermischt. Nach einigen Minuten erhält man eine gummielastische Masse. 30 Minuten nach Herstellung wird die Shore-Härte A zu 41 bestimmt. Die Konsistenz der angemischten Pasten beträgt gemäß ISO 4823 32 mm. 30 Minuten nach Herstellung beträgt der Randwinkel nach 3 Minuten Benetzungszeit 30°.

Der ausgehärtete Gummi ist mit dem Skalpell sehr gut beschneidbar. Lagerstabilität: Nach drei Tagen Lagerung trennt sich die ursprünglich homogene Paste in einen flüssigen, vorwiegend Polysiloxane enthaltenden, und einen festen, vorwiegend Füllstoff enthaltenden Teil auf. In diesem Zustand ist die Paste nicht mehr anwendbar. Die Abformmasse dieses Beispiels enthält zwar Diatomeenerde, jedoch in einer zu großen Menge.

## Patentansprüche

1. Monophasische additionsvernetzende Silikonabformmasse, enthaltend die Komponenten
(a) Organopolysiloxane mit mindestens zwei ungesättigten Gruppen im Molekül,
(b) Organohydrogenpolysiloxan mit mindestens 2 SiH-Gruppen im Molekül,
(c) gegebenenfalls Organopolysiloxan ohne reaktive Gruppen,
(d) Platin-Katalysator,
(e) Hydrophilierungsmittel,
(f) Diatomeenerde,
(g) Füllstoff, sowie
(h) gegebenenfalls weitere übliche Zusatz-, Hilfs- und Farbstoffe,
**dadurch gekennzeichnet, daß** die Menge an Komponente (f) 8 bis 25 Gew.-%, bezogen auf das Gewicht der Gesamtmasse der ausgehärteten Masse, beträgt, und die Masse eine nach DIN 53505 zu bestimmende Shore-Härte A von kleiner als 45 - gemessen nach 30 Minuten - und eine nach ISO 4823 zu bestimmende Konsistenz von 31 bis 39 mm aufweist.

2. Silikonabformmasse nach Anspruch 1, **dadurch gekennzeichnet, daß** die Menge an Komponente (f) 10 bis 20 Gew.-% beträgt.

3. Silikonabformmasse nach Anspruch 1, enthaltend
10-40 Gew.-% Komponenten (a) + (b)
0-40 Gew.-% Komponente (c)
0,00005-0.05 Gew.-% Komponente (d)
0,1-10 Gew.-% Komponente (e)
8-25 Gew.-% Komponente (f)
10-80 Gew.-% Komponente (g)
0-2 Gew.-% Komponente (h),
jeweils bezogen auf das Gesamtgewicht der Masse.

4. Silikonabformmasse nach den Ansprüchen 1 bis 3, enthaltend
15-25 Gew.-% Komponenten (a) + (b)
5-40 Gew.-% Komponente (c)
0,0002-0,04 Gew.-% Komponente (d)
0,2-2 Gew.-% Komponente (e)
10-20 Gew.-% Komponente (f)
30-60 Gew.-% Komponente (g)
0,1-1 Gew.-% Komponente (h)

5. Silikonabformmasse nach den Ansprüchen 1 bis 4, **dadurch gekennzeichnet, daß** das Mengenverhältnis der Komponente (a) zu Komponente (b) so gewählt wird, daß pro Mol an ungesättigter Doppelbindung der Komponente (a) 0,75 bis 5 Mol SiH-Gruppen der Komponente (b) vorliegen.

6. Silikonabformmasse nach den Ansprüchen 1 bis 5, **dadurch gekennzeichnet, daß** sie als Komponente (f) calcinierte Diatomeenerde enthält.

7. Silikonabformmasse nach den Ansprüchen 1 bis 6, **dadurch gekennzeichnet, daß** sie als Komponente (g) einen verstärkenden Füllstoff enthält.

8. Silikonabformmasse nach den Ansprüchen 1 bis 6, **dadurch gekennzeichnet, daß** sie als Komponente (g) ein Gemisch verschiedener Füllstoffe enthalten.

9. Silikonabformmasse nach Anspruch 8, **dadurch gekennzeichnet, daß** sie ein Gemisch aus einem verstärkenden Füllstoff und einem nichtverstärkenden Füllstoff enthält.

10. Silikonabformmasse nach den Ansprüchen 7 und 9, **dadurch gekennzeichnet, daß** sie als verstärkenden Füllstoff pyrogene Kieselsäure enthalten, welche vorzugsweise durch Oberflächenbehandlung hydrophobiert worden ist.

11. Silikonabformmasse nach den Ansprüchen 8 bis 10, dadurch gekenzeichnet, daß die verstärkenden Füllstoffe in einer Menge von 1 bis 10 Gew.-%, vorzugsweise 2 bis 5 Gew.-%, und die nichtverstärkenden Füllstoffe in einer Menge von 9 bis 70 Gew.-%, vorzugsweise 28 bis 55 Gew.-%, jeweils bezogen auf das Gewicht der gesamten Abformmasse vorliegen.

12. Silikonabformmasse nach den Ansprüchen 1 bis 11, **dadurch gekennzeichnet, daß** sie in Form einer Basispaste und einer davon räumlich getrennten Katalysatorpaste vorliegt, wobei die gesamte Komponente (b) in der Basispaste und die gesamte Komponente (d) in der Katalysatorpaste vorhanden sind und die übrigen Komponenten wahlweise in den beiden Pasten verteilt sind.

13. Silikonabformmasse nach den Ansprüchen 1 bis 12, **dadurch gekennzeichnet, daß** das Volumenverhältnis von Basispaste zu Katalysatorpaste 10:1 bis 1:10, vorzugsweise 1:1 oder 5:1, beträgt.

14. Verwendung einer monophasischen additionsvernetzenden Silikonabformmasse, enthaltend die Komponenten
(a) Organopolysiloxane mit mindestens zwei ungesättigten Gruppen im Molekül,
(b) Organohydrogenpolysiloxan mit mindestens 2 SiH-Gruppen im Molekül,
(c) gegebenenfalls Organopolysiloxan ohne reaktive Gruppen,
(d) Platin-Katalysator,
(e) Hydrophilierungsmittel,
(f) Diatomeenerde,
(g) Füllstoff, sowie
(h) gegebenenfalls weitere übliche Zusatz-, Hilfs- und Farbstoffe,
wobei die Menge an Komponente (f) 8 bis 25 Gew.-%, bezogen auf das Gewicht der Gesamtmasse der ausgehärteten Masse, beträgt, und die Masse eine nach DIN 53505 zu bestimmende Shore-Härte A von kleiner als 45 - gemessen nach 30 Minuten - und eine nach ISO 4823 zu bestimmende Konsistenz von 31 bis 39 mm aufweist, bei der Herstellung von provisorischen Brücken und Kronen.

## Claims

1. Monophase addition-cross-linking silicone impression material, comprising the components
(a) organopolysiloxanes with at least two unsaturated groups in the molecule,
(b) organohydrogenpolysiloxane with at least 2 SiH groups in the molecule,
(c) if appropriate organopolysiloxane without reactive groups,
(d) platinum catalyst
(e) hydrophilizing agent,
(f) diatomaceous earth,
(g) filler, and
(h) if appropriate further customary additives, auxiliaries and dyestuffs,
**characterized in that** the amount of component (f) is 8 to 25 wt.-%, based on the weight of the total mass of the cured material, and the material has a Shore A hardness, to be determined in accordance with DIN 53505, of less than 45 - measured after 30 minutes - and a consistency, to be determined in accordance with ISO 4823, of 31 to 39 mm.

2. Silicone impression material according to claim 1, **characterized in that** the amount of component (f) is 10 to 20 wt.-%.

3. Silicone impression material according to claim 1, comprising
10-40 wt.-% components (a) + (b)
0-40 wt.-% component (c)
0.00005-0.05 wt.-% component (d)
0.1-10 wt.-% component (e)
8-25 wt.-% component (f)
10-80 wt.-% component (g)
0-2 wt.-% component (h)
in each case based on the total weight of the material.

4. Silicone impression material according to claims 1 to 3, comprising
15-25 wt.-% components (a) + (b)
5-40 wt.-% component (c)
0.0002-0.04 wt.-% component (d)
0.2-2 wt.-% component (e)
10-20 wt.-% component (f)
30-60 wt.-% component (g)
0.1-1 wt.-% component (h)

5. Silicone impression material according to claims 1 to 4, **characterized in that** the ratio of amounts of component (a) to component (b) is chosen such that 0.75 mol to 5 mol SiH groups of component (b) are present per mol of unsaturated double bond of component (a).

6. Silicone impression material according to claims 1 to 5, **characterized in that** it comprises calcined diatomaceous earth as component (f).

7. Silicone impression material according to claims 1 to 6, **characterized in that** it comprises a reinforcing filler as component (g).

8. Silicone impression material according to claims 1 to 6, **characterized in that** it comprises a mixture of various fillers as component (g).

9. Silicone impression material according to claim 8, **characterized in that** it comprises a mixture of a reinforcing filler and a non-reinforcing filler.

10. Silicone impression material according to claims 7 and 9, **characterized in that** it comprises pyrogenic silicic acid, which has preferably been hydrophobized by surface treatment, as the reinforcing filler.

11. Silicone impression material according to claims 8 to 10, **characterized in that** the reinforcing fillers are present in an amount of 1 to 10 wt.-%, preferably 2 to 5 wt.-%, and the non-reinforcing fillers are present in an amount of 9 to 70 wt.-%, preferably 28 to 55 wt.-%, in each case based on the weight of the total impression material.

12. Silicone impression material according to claims 1 to 11, **characterized in that** it is in the form of a base paste and a catalyst paste spatially separated therefrom, the entire component (b) being present in the base paste and the entire component (d) being present in the catalyst paste and the other components optionally being distributed over the two pastes.

13. Silicone impression material according to claims 1 to 12, **characterized in that** the volume ratio of base paste to catalyst paste is 10:1 to 1:10, preferably 1:1 or 5:1.

14. Use of a monophase addition-cross-linking silicone impression material, comprising the components
(a) organopolysiloxanes with at least two unsaturated groups in the molecule,
(b) organohydrogenpolysiloxane with at least 2 SiH groups in the molecule,
(c) if appropriate organopolysiloxane without reactive groups,
(d) platinum catalyst
(e) hydrophilizing agent,
(f) diatomaceous earth,
(g) filler, and
(h) if appropriate further customary additives, auxiliaries and dyestuffs,
the amount of component (f) being 8 to 25 wt.-%, based on the weight of the total mass of the cured material, and the material having a Shore A hardness, to be determined in accordance with DIN 53505, of less than 45 - measured after 30 minutes - and a consistency, to be determined in accordance with ISO 4823, of 31 to 39 mm, in the production of temporary bridges and crowns.

## Revendications

1. Composition à monophase pour prise d'empreintes à base de silicones à réticulation par addition, contenant les composants :
(a) organopolysiloxanes ayant au moins deux groupes insaturés dans la molécule,
(b) organohydrogénopolysiloxane ayant au moins 2 groupes SiH dans la molécule,
(c) le cas échéant, un organopolysiloxane sans groupes réactifs,
(d) catalyseur au platine,
(e) agent conférant l'hydrophilie,
(f) terre à diatomées,
(g) charge, ainsi que
(h) le cas échéant, d'autres additifs, auxiliaires et colorants usuels,
**caractérisée en ce que** la quantité de composant (f) est de 8 à 25 % en poids par rapport au poids de la masse totale de la composition durcie, et **en ce que** la composition présente une dureté Shore A, à déterminer conformément à DIN 53505, inférieure à 45 (mesurée après 30 minutes) et une consistance, à déterminer conformément à ISO 4823, de 31 à 39 mm.

2. Composition pour prise d'empreintes à base de silicones selon la revendication 1, **caractérisée en ce que** la quantité de composant (f) est de 10 à 20 % en poids.

3. Composition pour prise d'empreintes à base de silicones selon la revendication 1, contenant
10-40 % en poids de composants (a) + (b)
0-40 % en poids de composant (c)
0,00005-0,05 % en poids de composant (d)
0,1-10 % en poids de composant (e)
8-25 % en poids de composant (f)
10-80 % en poids de composant (g)
0-2 % en poids de composant (h),
respectivement par rapport au poids total de la composition.

4. Composition pour prise d'empreintes à base de silicones selon les revendications 1 à 3, contenant
15-25 % en poids de composants (a) + (b)
5-40 % en poids de composant (c)
0,0002-0,04 % en poids de composant (d)
0,2-2 % en poids de composant (e)
10-20 % en poids de composant (f)
30-60 % en poids de composant (g)
0,1-1 % en poids de composant (h).

5. Composition pour prise d'empreintes à base de silicones selon les revendications 1 à 4, **caractérisée en ce qu'**on choisit le rapport quantitatif du composant (a) au composant (b) de façon à avoir de 0,75 à 5 moles de groupes SiH du composant (b) par mole de liaison double insaturée du composant (a).

6. Composition pour prise d'empreintes à base de silicones selon les revendications 1 à 5, **caractérisée en ce qu'**elle contient de la terre à diatomées calcinée en tant que composant (f).

7. Composition pour prise d'empreintes à base de silicones selon les revendications 1 à 6, **caractérisée en ce qu'**elle contient une charge renforçante en tant que composant (g).

8. Composition pour prise d'empreintes à base de silicones selon les revendications 1 à 6, **caractérisée en ce qu'**elle contient un mélange de diverses charges en tant que composant (g).

9. Composition pour prise d'empreintes à base de silicones selon la revendication 8, **caractérisée en ce qu'**elle contient un mélange formé d'une charge renforçante et d'une charge non renforçante.

10. Composition pour prise d'empreintes à basé de silicones selon les revendications 7 et 9, **caractérisée en ce qu'**elle contient de l'acide silicique pyrogène, en tant que charge renforçante, qu'on a rendu hydrophobe de préférence au moyen d'un traitement en surface.

11. Composition pour prise d'empreintes à base de silicones selon les revendications 8 à 10, **caractérisée en ce que** les charges renforçantes sont présentes en une quantité de 1 à 10 % en poids, de préférence de 2 à 5 % en poids, et les charges non renforçantes sont présentes en une quantité de 9 à 70 % en poids, de préférence de 28 à 55 % en poids, respectivement par rapport au poids de la composition totale pour prise d'empreintes.

12. Composition pour prise d'empreintes à base de silicones selon les revendications 1 à 11, **caractérisée en ce qu'**elle se présente sous la forme d'une pâte de base et d'une pâte de catalyseur spatialement séparée de celle-ci, la totalité du composant (b) étant présente dans la pâte de base et la totalité du composant (d) étant présente dans la pâte de catalyseur, et les composants restants étant répartis au choix entre les deux pâtes.

13. Composition pour prise d'empreintes à base de silicones selon les revendications 1 à 12, **caractérisée en ce que** le rapport en volume de la pâte de base à la pâte de catalyseur est de 10:1 à 1:10, de préférence de 1:1 ou de 5:1.

14. Utilisation d'une composition à monophase pour prise d'empreintes à base de silicones à réticulation par addition, contenant les composants :
(a) organopolysiloxanes ayant au moins deux groupes insaturés dans la molécule,
(b) organohydrogénopolysiloxane ayant au moins 2 groupes SiH dans la molécule,
(c) le cas échéant, un organopolysiloxane sans groupes réactifs,
(d) catalyseur au platine,
(e) agent conférant l'hydrophilie,
(f) terre à diatomées,
(g) charge, ainsi que
(h) le cas échéant, d'autres additifs, auxiliaires et colorants usuels,
la quantité de composant (f) étant de 8 à 25 % en poids par rapport au poids de la masse totale de la composition durcie, et la composition ayant une dureté Shore A, à déterminer conformément à DIN 53505, inférieure à 45 (mesurée après 30 minutes), et une consistance, à déterminer conformément à ISO 4823, de 31 à 39 mm, pour la fabrication de bridges et de couronnes provisoires.
